# FASCICULE DE BREVET EUROPEEN

(11) **EP 1 352 889 B1**
(45) Date de publication et mention de la délivrance du brevet: **30.03.2016**
(21) Numéro de dépôt: 03290842.8
(22) Date de dépôt: 04.04.2003
(51) Int. Cl.: C07C 45/50, C07B 41/06

(54) **Procédé d'hydroformylation mettant en oeuvre un catalyseur à base de cobalt dans un liquide ionique non-aqueux avec un recyclage du catalyseur amélioré**
Hydroformylierungsverfahren unter Verwendung eines Kobaltkatalysators in einer nicht-wässrigen ionischen Flüssigkeit mit Rückführung des Katalysators
Hydroformylation process using a cobalt catalyst in a non-aqueous ionic liquid with catalyst recycle

(30) Priorité: 11.04.2002 FR 0204563
(43) Date de publication de la demande: 15.10.2003
(73) Titulaire: Institut Français du Pétrole, 92852 Rueil-Malmaison Cedex (FR)
(72) Inventeur: Magna, Lionel, 92500 Rueil Malmaison (FR); Olivier Bourbigou, Hélène, 92500 Rueil Malmaison (FR); Saussine, Lucien, 78290 Croissy sur Seine (FR); Kruger-Tissot, Virginie, 92240 Malakoff (FR)

(56) Documents cités:
- EP-A- 1 106 595
- EP-A- 1 182 187
- C. C. BRASSE: "Ionic phosphine ligands with cobaltocenium backbone: novel ligands for the highly selective, biphasic, rhodium-catalyzed hydroformylation of 1-octene in ionic liquids" ORGANOMETALLICS, vol. 19, no. 19, 2000, pages 3818-3823, XP000993463

## Description

La présente invention concerne un procédé d'hydroformylation de composés oléfiniquement insaturés au moyen d'un catalyseur à base de cobalt mis en oeuvre en milieu biphasique, avec un recyclage du catalyseur amélioré. L'une des phases est constituée par un liquide ionique non-aqueux comprenant au moins un cation ammonium et/ou phosphonium quaternaire et/ou sulfonium Q⁺ et au moins un anion A⁻. Le catalyseur comprend au moins un complexe du cobalt.

L'hydroformylation des composés oléfiniques est une réaction de grande importance industrielle et la plupart des procédés ont recours à des catalyseurs homogènes dissous dans une phase organique constituée des réactifs, des produits et éventuellement d'un excès de ligand, si bien que l'on rencontre des difficultés pour séparer et récupérer le catalyseur, en particulier quand celui-ci est employé en relativement grande quantité, comme c'est le cas avec les catalyseurs à base de cobalt.

Une solution en vue de résoudre ce problème a été évoquée par Bartik et al. : Organometallics (1993) 12 164-170, J. Organometal. Chem. (1994) 480 15-21, ainsi que par Beller et al. : J. Molecular Catal. A : Chemical (1999) 143 31-39. Elle consiste à effectuer l'hydroformylation en présence d'une solution aqueuse contenant un complexe du cobalt qui est rendu hydrosoluble grâce à la présence d'un ligand phosphine-sulfonate, tel que le sel de sodium de la triphénylphosphine trisulfonée ou d'une tris-(alkylphényl)-phosphine trisulfonée. La demande internationale WO-A-97/00 132 décrit des clusters de cobalt substitués par des groupements trialkoxysilylméthyle qui les rendent solubles dans l'eau. De cette manière, la phase organique contenant les aldéhydes est aisément séparée de la phase aqueuse contenant le catalyseur.

En dépit du grand intérêt industriel de ces techniques pour l'hydroformylation des composés oléfiniques, ces systèmes à deux phases souffrent du manque de solubilité des oléfines dans l'eau, ce qui conduit à des vitesses de réaction relativement faibles qui les rendent inapplicables pour les oléfines à longue chaîne.

Par ailleurs, a été décrit dans le brevet US-A-3 565 823 une technique consistant à disperser un composé d'un métal de transition dans un sel d'étain ou de germanium et d'un ammonium ou d'un phosphonium quaternaire, de formule (R¹R²R³R⁴Z)YX₃ dans laquelle R¹, R², R³ et R⁴ sont des restes hydrocarbyles ayant jusqu'à 18 atomes de carbone, Z est l'azote ou le phosphore, Y l'étain ou le germanium et X un halogène, chlore ou brome. Dans le brevet US-A-3 832 391, a été décrit un procédé de carbonylation des oléfines par la même composition. Les compositions précédentes présentent le désavantage d'avoir un point de fusion relativement élevé, par exemple supérieur à 90°C, ce qui complique la manipulation des solutions de catalyseur et des produits de réaction.

La publication de Brasse et al. (Organometa//ics, vol, 19, n°19 p 3818-3823) décrit un procédé d'hydroformylation mettant en oeuvre un catalyseur à base de rhodium et utilisant comme ligands des sels de cobaltocènes substitués par des phosphines, dans des liquides ioniques.

La demande EP 1 106 595 décrit un procédé d'hydroformylation mettant en oeuvre un catalyseur à base de cobalt et/ou de rhodium coordiné par au moins un ligand azoté, en milieu biphasique.

La demande EP 1 182 187 décrit un procédé d'hydroformylation mettant en oeuvre un catalyseur à base de cobalt et/ou de rhodium coordiné par au moins un ligand azoté, en milieu biphasique, dans lequel le ligand coordiné au métal porte une fonction ionique Q'+A'-, de même nature chimique que les constituants Q+A- du solvant ionique non-aqueux.

Il a été décrit dans le brevet US-A-5 874 638 de la demanderesse que l'on peut à la fois bénéficier des avantages d'une mise en oeuvre à deux phases tout en évitant les inconvénients liés d'une part à l'utilisation de l'eau et d'autre part à l'emploi de composés à point de fusion élevé, en dissolvant certains composés catalytiques des métaux de transition des groupes 8, 9 et 10, connus pour catalyser l'hydroformylation, dans des liquides ioniques non-aqueux qui sont constitués par des sels organiques-inorganiques liquides à température ambiante.

Cependant, lorsque le catalyseur comprend un sel ou un complexe du cobalt, il est très difficile d'empêcher la formation, au moins en partie, de dicobalt-octacarbonyle et/ou d'hydrure de cobalt-tétracarbonyle dans les conditions de la réaction d'hydroformylation. Ces deux composés étant bien solubles dans la phase organique de réaction constituée au moins par le réactif oléfinique et les aldéhydes produits, le recyclage du cobalt au moyen de la phase liquide ionique non-aqueux n'est que partiel, ce qui entraîne des pertes de catalyseur.

Il a maintenant été trouvé que, dans la réaction d'hydroformylation catalysée par des complexes du cobalt mis en oeuvre dans un liquide ionique non-aqueux comprenant au moins un cation ammonium et/ou phosphonium et/ou sulfonium Q⁺ et au moins un anion A⁻, liquide à une température inférieure à 90 °C, le recyclage du métal dans le liquide ionique est grandement amélioré par l'utilisation d'un ligand choisi dans le groupe formé par les bases de Lewis et par la mise en oeuvre d'une étape de dépressurisation intermédiaire entre l'étape de réaction sous pression et l'étape de séparation des phases par décantation. A la fin de cette étape de dépressurisation, la phase organique est séparée dans l'étape de décantation, et la phase liquide ionique non-aqueux contenant le catalyseur peut être réutilisée.

Plus précisément, l'invention a pour objet un procédé en continu pour l'hydroformylation en phase liquide des composés oléfiniquement insaturés comprenant une étape de réaction sous pression effectuée en présence d'au moins un liquide ionique non-aqueux comprenant au moins un sel de formule générale Q+A-, dans laquelle Q+ représente un ammonium quaternaire et/ou un phosphonium quaternaire et/ou sulfonium, et A représente un anion, et une étape de décantation des produits finaux, caractérisé en ce que le catalyseur comprend au moins un complexe du cobalt avec au moins un ligand, et en ce que l'on met en oeuvre une étape de dépressurisation intermédiaire entre l'étape de réaction et l'étape de décantation, le contact entre les deux phases liquides étant maintenu dans l'étape de dépressurisation grâce à une agitation mécanique, ledit ligand étant choisi dans le groupe formé par
- les ligands oxygénés, choisis dans le groupe formé par les alcools, les phénols, les éthers, les cétones, les acétals
- les ligands soufrés, choisis dans le groupe formé par les thiols, les thiophénols, les thioéthers et les bisulfures
- les ligands azotés choisis dans le groupe formé par les monoamines, les di-, tri- et polyamines, les imines, les diimines, les pyridines, les bipyridines, les imidazoles, les pyrroles et les pyrazoles, et
- les ligands phosphorés, choisis dans le groupe formé par les phosphines, les poly-phosphines, les oxydes de phosphines et les phosphites,
les dits ligands étant substitués ou non par des groupements fonctionnels ioniques comme par exemple les sulfonates, les carboxylates, les phosphates, les ammoniums et les phosphoniums.

Sans vouloir être lié par une quelconque théorie, on peut penser que dans les conditions de la réaction d'hydroformylation sous pression de gaz de synthèse, le catalyseur est présent en grande partie sous forme des complexes Co₂(CO)₈, Co₂(CO)₆L₂, HCo(CO)₄, et HCo(CO)₃L, où L est le ligand base de Lewis, et que dans l'étape de dépressurisation avant décantation, la présence du ligand L basique favorise la formation de complexes du cobalt ioniques tels que par exemple (CoL₆)⁺⁺[Co(CO)₄⁻]₂ qui ont une grande affinité pour la phase contenant le liquide ionique non-aqueux.

Le liquide ionique non-aqueux est choisi dans le groupe formé par les sels liquides qui ont pour formule générale Q⁺ A- dans laquelle Q⁺ représente un ammonium quaternaire et/ou un phosphonium quaternaire et/ou un sulfonium et A- représente tout anion susceptible de former un sel liquide à basse température, c'est-à-dire en dessous de 90 °C et avantageusement d'au plus 85 °C, et de préférence en dessous de 50 °C. Les anions A⁻ préférés sont les ions nitrate, sulfate, phosphate, acétate, halogénoacétates, tétrafluoroborate, tétrachoroborate, tétraalkylborate, tétraarylborate, hexafluorophosphate, hexafluoroantimonate, fluorosulfonate, alkylsulfonates, perfluoroalkylsulfonates, bis(perfluoroalkylsulfonyl)amidures, arènesulfonates, ces derniers éventuellement substitués par des groupements halogénés ou halogénoalkyles.

Les ammonium et/ou phosphonium quaternaires Q⁺ répondent de préférence aux formules générales NR¹R²R³R⁴⁺ et PR¹R²R³R⁴⁺, ou aux formules générales R¹R²N=CR³R⁴⁺ et R¹R²P=CR³R⁴⁺ où R¹, R², R³ et R⁴, identiques ou différents, représentent l'hydrogène (à l'exception du cation NH₄⁺ pour NR¹R²R³R⁴⁺), de préférence un seul substituant représentant l'hydrogène, ou des restes hydrocarbyles ayant de 1 à 30 atomes de carbone, par exemple des groupements alkyles, saturés ou non saturés, cycloalkyles ou aromatiques, aryles ou aralkyles, éventuellement substitués, comprenant de 1 à 30 atomes de carbone. Les ammonium et/ou phosphonium peuvent également être dérivés d'hétérocycles azotés et/ou phosphorés comportant 1, 2 ou 3 atomes d'azote et/ou de phosphore, dans lesquelles les cycles sont constitués de 4 à 10 atomes, de préférence 5 à 6 atomes.

Les cations ammoniums et phosphoniums quaternaires peuvent également répondre respectivement aux formules :

R¹R²+N=CR³-R⁵-R³C=N+R¹R²

et

R¹R²+P=CR³-R⁵-R³C=P+R¹R²

dans lesquelles R¹, R² et R³, identiques ou différents, sont définis comme précédemment et R⁵ représente un reste alkylène ou phénylène. Parmi les groupements R¹, R², R³ et R⁴ on mentionnera les radicaux méthyle, éthyle, propyle, isopropyle, butyle, butyle secondaire, butyle tertiaire, amyle, méthylène, éthylidène, phényle ou benzyle; R⁵ pourra être un groupement méthylène, éthylène, propylène ou phénylène.

Le cation ammonium et/ou phosphonium Q⁺ est choisi de préférence dans le groupe formé par le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, le diéthyl-pyrazolium, le N-butyl-N-méthylpyrrolidinium, le triméthylphényl-ammonium, le tétrabutylphosphonium et le tributyl-tétradécyl-phosphonium.

Les cations sulfonium des sels utilisables selon l'invention peuvent répondre à la formule générale SR¹R²R³⁺, dans laquelle R¹, R² et R³, identiques ou différents, représentent chacun un reste hydrocarbyle ayant de 1 à 12 atomes de carbone, par exemple un groupement alkyle, saturé ou non saturé, cycloalkyle ou aromatique, aryle, alkaryle ou aralkyle, comprenant de 1 à 12 atomes de carbone.

A titre d'exemples des sels utilisables selon l'invention, on peut citer l'hexafluorophosphate de N-butyl-pyridinium, le tétrafluoroborate de N-éthyl-pyridinium, le fluorosulfonate de pyridinium, le tétrafluoroborate de butyl-3-méthyl-1-imidazolium, l'hexafluoro-antimonate de butyl-3-méthyl-1-imidazolium, l'hexafluorophosphate de butyl-3-méthyl-1-imidazolium, le trifluoroacétate de butyl-3-méthyl-1-imidazolium, le trifluorométhylsulfonate de butyl-3-méthyl-1-imidazolium, le bis(trifluorométhylsulfonyl)amidure de butyl-3-méthyl-1-imidazolium, l'hexafluorophosphate de triméthyl-phénylammonium et le tétrafluoroborate de tétrabutylphosphonium. Ces sels peuvent être utilisés seuls ou en mélange.

Les composés du cobalt précurseurs du catalyseur sont choisis dans le groupe formé par les sels de cobalt, comme les acétylacétonates, les carboxylates et en particulier le formiate ou l'acétate, et les complexes carbonyle, comme le dicobalt-octacarbonyle, l'hydrure de cobalt-tétracarbonyle et les clusters carbonyles. Le choix du composé précurseur du cobalt n'est pas critique, mais on préfère en général éviter les halogénures.

Le ligand basique de Lewis est choisi dans le groupe formé par les ligands oxygénés, les ligands soufrés, les ligands azotés et les ligands phosphorés, substitués ou non par des groupements fonctionnels ioniques comme par exemple les sulfonates, les carboxylates, les phosphates, les ammoniums et les phosphoniums.

Les ligands oxygénés sont choisis plus particulièrement dans le groupe formé par les alcools, les phénols, les éthers, les cétones et les acétals. On peut citer à titre d'exemples non limitatifs le méthanol, l'éthanol, le phénol, le diéthyléther, le dibutyléther, le diphényléther, le tétrahydrofurane, le dioxane-1,4, le dioxolane-1,3, le glyme, le diglyme, l'acétone, la méthyéthylcétone, l'acéto-phénone, le méthylal, le diméthoxy-2,2 propane et le di(éthyl-2 hexyloxy)-2,2 propane.

Les ligands soufrés sont choisis plus particulièrement dans le groupe formé par les thiols, les thiophénols, les thioéthers et les disulfures. On peut citer à titre d'exemples non limitatifs le méthanethiol, l'éthanethiol, le thiophénol, le diéthyl-sulfure, le diméthyldisulfure et le tétrahydrothiophène.

Les ligands azotés sont choisis plus particulièrement dans le groupe formé par les monoamines, les di-, tri- et polyamines, les imines, les diimines, les pyridines, les bipyridines, les imidazoles, les pyrroles, les pyrazoles. On peut citer à titre d'exemples non limitatifs la méthylamine, la triméthylamine, la triéthylamine, l'éthylènediamine, la diéthylènetriamine, le diazabicyclooctane, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, la pyridine, la 2-picoline, la 4-picoline, la t-butyl-2-pyridine, la di(t-butyl)-2,6-pyridine, la 2,2'-bipyridine, l'imidazole, le N-méthylimidazole, le N-butylimidazole, le pyrrole, le N-méthylpyrrole et le diméthyl-2,5-pyrrole.

Les ligands phosphorés sont choisis plus particulièrement dans le groupe formé par les phosphines, les polyphosphines, les oxydes de phosphines, les phosphites. On peut citer à titre d'exemples non limitatifs la tributylphosphine, la triisopropylphosphine, la tricyclohexylphosphine, la triphénylphosphine, la tris(o-tolyl)phosphine, le bis(diphénylphosphino)éthane, l'oxyde de trioctylphosphine, l'oxyde de triphénylphosphine et le triphénylphosphite.

Les ligands préférés sont choisis dans le groupe formé par les pyridines et les phosphines substituées par des groupements fonctionnels ioniques comme par exemple les sulfonates, les carboxylates, les phosphates, les ammoniums, les phosphoniums. On peut citer à titre d'exemples non limitatifs :
- le ligand 1-(4-pyridyl)-2-(dicyclopentylméthylphosphonium)-éthane tétrafluoroborate (**1**),
- le ligand 1-(N-imidazolyl)-2-(dicyclopentyl-méthyl-phosphonium)-éthane tétrafluoroborate (**2**),
- le ligand 1-(diphénylphosphino)-2-(4-N-méthyl-pyridinium)-éthane hexafluorophosphate (**3**),
- le ligand 1-(dicyclopentylphosphino)-2-(3-méthyl-1 imidazolium)-éthane hexafluorophosphate (**4**),
- le ligand triphénylphosphine trisulfonate de tétrabutylammonium,
- le ligand triphénylphosphine trisulfonate de sodium (ou TPPTS),
- le ligand triphénylphosphine monosulfonate de sodium (ou TPPMS),
- le ligand (méthyl-3-imidazolium-1-yl hexafluorophosphate)-2-pyridine (**5)** et
- le ligand bis(méthyl-3-imidazolium-1-yl hexafluorophosphate)-2,6-pyridine (**6**). 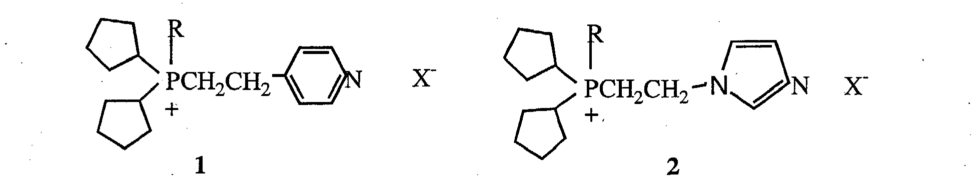 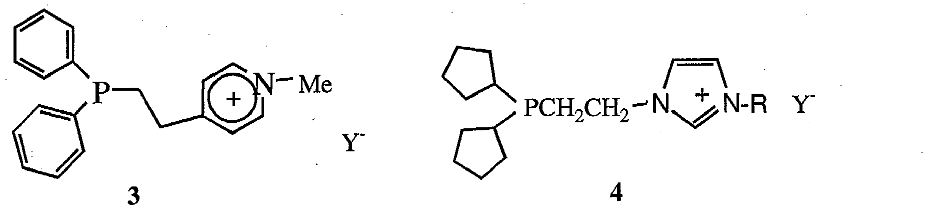 R=Me, X=BF₄, Y=PF₆

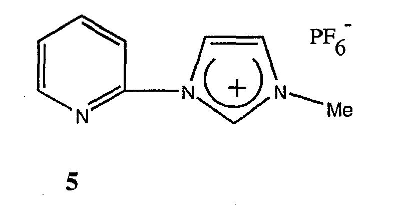

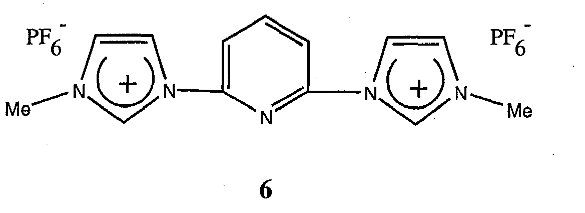

La composition catalytique est obtenue par mélange, d'une manière quelconque, du liquide ionique avec le composé du cobalt et le ligand. On peut également dissoudre préalablement le composé du métal de transition et/ou le ligand dans un solvant organique.

Le complexe entre le précurseur de cobalt et le ligand peut être préparé préalablement à la réaction par mélange du précurseur de cobalt avec le ligand dans un solvant convenable, par exemple un solvant organique ou le liquide ionique non-aqueux qui sera utilisé ensuite dans la réaction catalytique. Le complexe peut aussi être préparé in situ par mélange du précurseur de cobalt et du ligand directement dans le réacteur d'hydroformylation. Il est également possible d'injecter le ligand seulement dans l'étape de dépressurisation suivant la réaction.

La concentration du complexe de cobalt dans le liquide ionique non-aqueux n'est pas critique. Elle est avantageusement comprise entre 0,1 mmole par litre de liquide ionique et 5 moles par litre, de préférence entre 1 mmole et 1 mole par litre, et, de manière encore plus préférée, entre 10 et 500 mmoles par litre. Le rapport molaire entre le ligand et le composé du cobalt est compris entre 0,1:1 et 500:1, de préférence entre 1:1 et 100:1.

Les composés oléfiniquement insaturés susceptibles d'être hydroformylés sont choisis dans le groupe formé par les monooléfines, les dioléfines, en particulier les dioléfines conjuguées, et les composés oléfiniques comportant un ou plusieurs hétéroatomes, notamment dans des groupements insaturés comme la fonction cétone ou acide carboxylique. A titre d'exemples non limitatifs, on peut citer l'hydroformylation des pentènes en hexanal et méthylpentanal, des hexènes en heptanals et isoheptanals, des isooctènes en isononanals, des isodécènes en iso-undécanals, et celle des coupes C₁₁ à C₁₆ oléfiniques en aldéhydes C₁₂ à C₁₇. Ces composés oléfiniques peuvent être mis en oeuvre purs ou dilués par des hydrocarbures saturés ou insaturés.

Le rapport des pressions partielles de l'hydrogène au monoxyde de carbone utilisé dans le milieu réactionnel pour l'hydroformylation peut être de 10:1 à 1:10, de préférence dans un rapport 1:1, mais tout autre rapport peut être utilisé selon la mise en oeuvre du procédé.

La température à laquelle se fera l'hydroformylation sera comprise entre 30 °C et 200 °C, avantageusement la température est inférieure à 180 °C, de préférence entre 50 °C et 150 °C. La pression peut être comprise entre 1 MPa et 20 MPa, de préférence entre 2 MPa et 15 MPa. Des conditions particulières sont par exemple une température de 95 °C et une pression de 6,5 MPa ou une température de 140 °C et une pression de 10 MPa.

La réaction catalytique d'hydroformylation des composés insaturés peut être conduite en continu avec un ou plusieurs étages de réaction. Dans une mise en oeuvre en continu, l'effluent du réacteur sous pression est transféré dans une enceinte où il est dépressurisé jusqu'à une pression inférieure à 1 MPa et de préférence à la pression atmosphérique, à une température au plus égale à 150 °C et de préférence inférieure à 60 °C. Le contact entre les deux phases liquides peut être maintenu dans cette étape grâce à une agitation mécanique, ou à tout autre moyen convenable. Le temps de contact dans l'enceinte de dépressurisation doit être suffisant pour assurer au mieux le transfert du catalyseur dans la phase liquide ionique non-aqueux.

A la sortie de l'enceinte de dépressurisation, la phase organique contenant les produits de réaction est séparée avantageusement par simple décantation de la phase liquide ionique non-aqueux contenant la presque totalité du catalyseur. Cette phase liquide ionique qui contient le catalyseur, est, au moins en partie, retournée au réacteur, l'autre partie pouvant être traitée pour éliminer des résidus de décomposition du catalyseur.

L'invention a également pour objet une installation pour la mise en oeuvre du procédé d'hydroformylation tel que défini dans la description qui précède, ladite installation comportant :
- au moins un réacteur A1 ;
- au moins une enceinte de dépressurisation (« dépressuriseur ») B1 ;
- et au moins un décanteur B2 pour la décantation de la phase polaire contenant au moins le solvant ionique non-aqueux ionique contenant au moins le catalyseur qui est recyclé au réacteur A1 ;
ainsi que :
- au moins une conduite 1 pour l'introduction de la charge à hydroformyler et du mélange monoxyde de carbone/hydrogène ;
- au moins une conduite 2 pour le transfert de l'effluent du réacteur vers le dépressuriseur B1 ;
- au moins une conduite 3 pour envoyer vers le décanteur B2 le mélange de l'effluent organique et du solvant ionique contenu dans le dépressuriseur B1 ;
- au moins une conduite 6 pour renvoyer vers le réacteur A1 les gaz issus du dépressuriseur B1 ;
- au moins une conduite 5 permettant de renvoyer dans le réacteur A1 la phase polaire contenant au moins le liquide ionique et le catalyseur séparée dans B2 ;
- au moins une conduite 7 permettant de soutirer du décanteur B2 les produits bruts de la réaction.

L'installation comprend en outre :
- dans la section de séparation, au moins une colonne A2 pour la séparation des produits bruts de la réaction et du composé oléfiniquement insaturé à hydroformyler qui n'a pas réagi ;
ainsi que :
- au moins une conduite 4 pour recycler vers le réacteur A1 le composé oléfiniquement insaturé à hydroformyler qui n'a pas réagi séparé dans la colonne A2 ;
- au moins une conduite 8 permettant d'envoyer les produits sortant en pied de la colonne A2 dans la suite du train de fractionnement des produits.

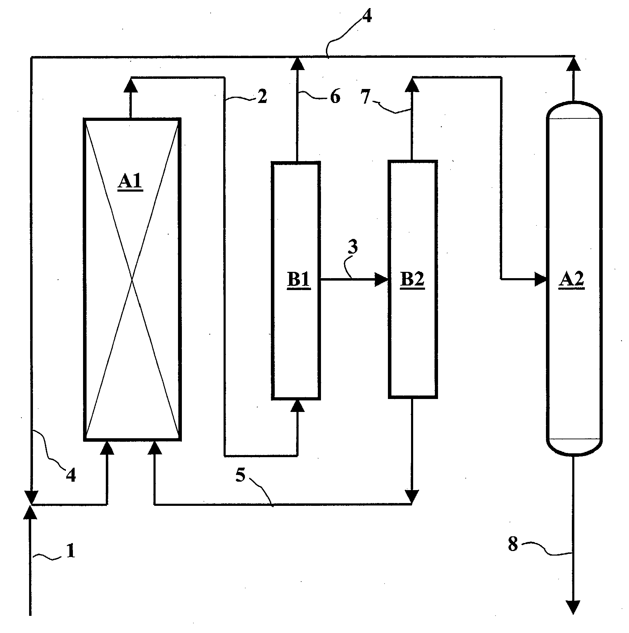

On comprendra mieux le procédé et l'installation de l'invention à partir de la description qui en est faite ci-après, en liaison avec la Figure 1.

Selon la Figure 1, la réaction est réalisée dans le réacteur A1 en présence de la charge à hydroformyler, qui peut être introduite par la ligne 1, du (ou des) composé(s) de métal de transition, du ligand base de Lewis (qui optionnellement peut être introduit en mélange avec le (ou les) composé(s) des métaux de transition), de monoxyde de carbone et d'hydrogène, qui peuvent être introduits par la ligne 1, et en présence d'au moins un liquide ionique non-aqueux. Le liquide ionique peut être introduit dans le réacteur au début de la réaction. Optionnellement, du liquide ionique frais peut être injecté dans le réacteur A1 au cours de la réaction et du liquide ionique usé soutiré de A1 (les moyens d'injection et de soutirage du liquide ionique ne sont pas indiqués dans la Figure 1).

La chaleur de réaction est éliminée par les techniques connues de l'homme du métier, qui ne sont pas représentées sur la Figure 1.

A la sortie de la section réactionnelle, l'effluent du réacteur est envoyé, par la ligne 2, dans au moins un dépressuriseur B1 dans lequel la pression est abaissée. Une agitation peut être maintenue dans B1, soit mécaniquement, soit par tout autre moyen convenable. Les gaz libérés par la dépressurisation s'échappent par la ligne 6 et sont renvoyés vers le réacteur A1 après avoir été recomprimés.

L'effluent du dépressuriseur B1 est envoyé dans le décanteur B2 par la ligne 3. Dans ce décanteur B2, la phase polaire qui contient au moins le liquide ionique et le catalyseur est séparée du mélange des produits et du solvant organique et est renvoyée au réacteur A1 par la ligne 5.

La phase organique séparée dans le décanteur B2 est envoyée dans une colonne de distillation A2 par la ligne 7. Dans la colonne A2, on sépare en tête le composé oléfiniquement insaturé à hydroformyler qui n'a pas réagi. Il est recyclé au réacteur A1 par la ligne 4. Les produits bruts de la réaction recueillis en pied de A2 sont envoyés dans un train de fractionnement spécifique (non représenté) par la ligne 8.

Les exemples suivants illustrent l'invention sans en limiter la portée.

### EXEMPLE 1 (à titre de référence)

La réaction d'hydroformylation est conduite dans un autoclave en acier inoxydable d'une contenance de 300 mL, muni d'une double enveloppe permettant la régulation de la température par circulation d'un fluide caloporteur, et équipé d'une agitation mécanique efficace avec pales et contre-pales. Dans cet autoclave purgé au préalable de l'air et de l'humidité et placé sous pression atmosphérique du gaz de synthèse hydrogène-monoxyde de carbone (1/1 molaire), on introduit 0,4 g de dicobalt-octacarbonyle (soit 2,3 mmole de cobalt), 1 équivalent molaire de pyridine désaérée et distillée (ligand base de Lewis), 10 mL de bis(trifluorométhylsulfonyl)amidure de butyl-3-méthyl-1-imidazolium (liquide ionique non-aqueux), 30 mL d'heptane et 30 mL d'hexène-1. On porte la pression du gaz de synthèse à 10 MPa et la température à 140 °C et on met l'agitation en route. Après 3,5 heures de réaction, on ferme l'arrivée du gaz de synthèse et on laisse refroidir le réacteur jusqu'à 25 °C. En maintenant l'agitation, on relâche alors lentement la pression jusqu'à la pression atmosphérique. L'agitation est arrêtée et le mélange réactionnel est laissé à décanter pendant une nuit. Après soutirage hors de l'autoclave, la phase organique supérieure est légèrement colorée.

L'analyse par chromatographie en phase gazeuse des deux phases après flash et la pesée des résidus non flashés permettent d'établir le bilan matière de la réaction. La conversion de l'hexène-1 est de 99,5 % en poids. La sélectivité pour les aldéhydes en C₇ est de 77,6 % et le rapport n/iso (n-heptanal / isoheptanals) est égal à 1,2. La phase organique contient 7,3 % de la totalité du cobalt engagé dans la réaction.

### EXEMPLE 2 (à titre de référence)

La réaction d'hydroformylation est conduite dans le même appareillage et selon le même mode opératoire que décrit dans l'Exemple 1, à ceci près que le dégazage de l'autoclave est réalisé en absence d'agitation. Après 4 heures de réaction, on soutire hors de l'autoclave. La phase organique supérieure est légèrement colorée.

La conversion de l'hexène-1 est de 97,9 % en poids. La sélectivité pour les aldéhydes en C₇ est de 85,9 % et le rapport n/iso (n-heptanal / isoheptanals) est égal à 1,6. La teneur en cobalt métal de la phase organique est de 440 ppm (parties par million en poids), ce qui correspond à 12,8 % du cobalt engagé dans la réaction.

### EXEMPLE 3 (à titre de référence)

La réaction d'hydroformylation est conduite dans le même appareillage et selon le même mode opératoire que décrit dans l'Exemple 1, à ceci près que le ligand est le bis(méthyl-3-imidazolium-1-yl hexafluorophosphate)-2,6-pyridine (1 équivalent molaire par rapport au cobalt), et que le liquide ionique non-aqueux est l'hexafluorophosphate de butyl-3-méthyl-1-imidazolium. Après 3 heures de réaction, on soutire hors de l'autoclave. La phase organique supérieure est pratiquement incolore.

La conversion de l'hexène-1 est de 97,7 % en poids. La sélectivité pour les aldéhydes en C₇ est de 55,3 % et le rapport n/iso (n-heptanal / isoheptanals) est égal à 0,9. La teneur en cobalt métal de la phase organique est de 15 ppm (parties par million en poids), ce qui correspond à 0,5 % du cobalt engagé dans la réaction.

### EXEMPLE 4 (à titre de référence)

La réaction d'hydroformylation est conduite dans le même appareillage et selon le même mode opératoire que décrit dans l'Exemple 1, à ceci près que le ligand est la triphénylphosphine monosulfonate de sodium (1 équivalent molaire pour le cobalt). Après 6 heures de réaction, on soutire hors de l'autoclave. La phase organique supérieure est légèrement colorée.

La conversion de l'hexène-1 est de 96,8 % en poids. La sélectivité pour les aldéhydes en C₇ est de 73,7 % et le rapport n/iso (n-heptanal / isoheptanals) est égal à 1,4. La teneur en cobalt métal de la phase organique est de 180 ppm (parties par million en poids), ce qui correspond à 6 % du cobalt engagé dans la réaction.

### EXEMPLE 5 (comparatif)

La réaction d'hydroformylation est conduite dans le même appareillage et selon le même mode opératoire que décrit dans l'Exemple 1, à ceci près qu'on a omis d'introduire un ligand. Après 6 heures de réaction, on soutire hors de l'autoclave. La phase organique supérieure est fortement colorée en brun foncé.

La conversion de l'hexène-1 est de 99,6 % en poids. La sélectivité pour les aldéhydes en C₇ est de 18,5 % et le rapport n/iso (n-heptanal / isoheptanals) est égal à 0,4. La phase organique contient 90 % du cobalt engagé dans la réaction.

Cet exemple comparatif montre qu'en l'absence de ligand, le recyclage du cobalt dans le liquide ionique non-aqueux est compromis par la solubilité des complexes carbonyle dans la phase organique.

### EXEMPLES 6 À 10 (comparatifs)

Le même comportement caractéristique de l'absence de ligand que dans l'Exemple 5 a été vérifié sur une large gamme de liquides ioniques :
- Exemple 6 : tétrafluoroborate de butyl-3-méthyl-1-imidazolium ;
- Exemple 7 : bis(trifluorométhylsulfonyl)amidure de butyl-3-méthyl-1-imida-zolium ;
- Exemple 8: hexafluorophosphate de butyl-3-méthyl-2-méthyl-1-imidazolium ;
- Exemple 9 : tétrafluoroborate de butyl-3-méthyl-2-méthyl-1-imidazolium ; et
- Exemple 10 :bis(trifluorométhylsulfonyl)amidure de butyl-3-méthyl-2-méthyl-1-imidazolium.

Dans ces exemples menés sans ligands, avec une même conversion et une même sélectivité que dans l'Exemple 5, on a observé des teneurs en cobalt métal dans la phase organique correspondant à des proportions de 70 à 100 % du cobalt engagé dans la réaction.

## Revendications

1. Procédé en continu pour l'hydroformylation en phase liquide des composés oléfiniquement insaturés comprenant une étape de réaction sous pression effectuée en présence d'au moins un liquide ionique non-aqueux comprenant au moins un sel de formule générale Q⁺A⁻, dans laquelle Q⁺ représente un ammonium quaternaire et/ou un phosphonium quaternaire et/ou sulfonium, et A⁻ représente un anion, et une étape de décantation des produits finaux, **caractérisé en ce que** le catalyseur comprend au moins un complexe du cobalt avec au moins un ligand, et **en ce que** l'on met en oeuvre une étape de dépressurisation intermédiaire entre l'étape de réaction et l'étape de décantation, le contact entre les deux phases liquides étant maintenu dans l'étape de dépressurisation grâce à une agitation mécanique, ledit ligand étant choisi dans le groupe formé par
- les ligands oxygénés, choisis dans le groupe formé par les alcools, les phénols, les éthers, les cétones, les acétals
- les ligands soufrés, choisis dans le groupe formé par les thiols, les thiophénols, les thioéthers et les disulfures
- les ligands azotés choisis dans le groupe formé par les monoamines, les di-, tri- et polyamines, les imines, les diimines, les pyridines, les bipyridines, les imidazoles, les pyrroles et les pyrazoles, et
- les ligands phosphorés, choisis dans le groupe formé par les phosphines, les poly-phosphines, les oxydes de phosphines et les phosphites,
les dits ligands étant substitués ou non par des groupements fonctionnels ioniques comme par exemple les sulfonates, les carboxylates, les phosphates, les ammoniums et les phosphoniums.

2. Procédé selon la revendication 1 dans lequel l'effluent du réacteur sous pression est transféré dans une enceinte où il est dépressurisé jusqu'à une pression inférieure à 1 MPa et de préférence à la pression atmosphérique, à une température au plus égale à 150°C et de préférence inférieure à 60°C.

3. Procédé selon l'une des revendications 1 ou 2, **caractérisé en ce que** le liquide ionique non-aqueux est choisi dans le groupe formé par les sels liquides qui ont pour formule générale Q⁺ A⁻ dans laquelle Q⁺ représente un ammonium quaternaire et/ou un phosphonium quaternaire et/ou sulfonium et A⁻ représente tout anion susceptible de former un sel liquide à basse température, c'est-à-dire en dessous de 90 °C.

4. Procédé selon la revendication 3, **caractérisé en ce que** les anions A- sont choisis parmi les ions nitrate, sulfate, phosphate, acétate, halogénoacétates, tétrafluoroborate, tétrachoroborate, hexafluorophosphate, hexafluoroantimonate, fluorosulfonate, alkylsulfonates, perfluoroalkylsulfonates, bis(perfluoroalkylsulfonyl)amidures, arènesulfonates, et arènesulfonates substitués par des groupements halogènes ou halogénoalkyles.

5. Procédé selon la revendication 3, **caractérisé en ce que** les cations ammoniums et/ou phosphoniums quaternaires répondent aux formules générales NR¹R²R³R⁴⁺ et PR¹R²R³R⁴⁺, ou aux formules générales R¹R²N=CR³R⁴⁺ et R¹R²P=CR³R⁴⁺ dans lesquelles R¹, R², R³ et R⁴, identiques ou différents, représentent l'hydrogène à l'exception du cation NH₄⁺ et de préférence un seul substituant peut représenter l'hydrogène, ou des restes hydrocarbyles ayant de 1 à 30 atomes de carbone.

6. Procédé selon la revendication 3, **caractérisé en ce que** les cations ammoniums et/ou phosphoniums sont dérivés d'hétérocycles azotés et/ou phosphorés comportant 1, 2 ou 3 atomes d'azote et/ou de phosphore, dans lesquelles les cycles sont constitués de 4 à 10 atomes.

7. Procédé selon la revendication 3, **caractérisé en ce que** les cations ammoniums et phosphoniums quaternaires répondent respectivement aux formules :
R¹R²⁺N=CR³-R⁵-R³C=N⁺R¹R²
et
R¹R²+P=CR³-R⁵-R³C=P⁺R¹R²
dans laquelle R¹, R² et R³, identiques ou différents sont définis comme précédemment et R⁵ représente un reste alkylène ou phénylène.

8. Procédé selon la revendication 3, **caractérisé en ce que** les cations ammoniums et/ou phosphoniums sont choisis dans le groupe formé par le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, le diéthyl-pyrazolium, le N-butyl-N-méthylpyrrolidinium, le triméthylphényl-ammonium, le tétrabutylphosphonium et le tributyl-tétradécyl-phosphonium.

9. Procédé selon la revendication 3, **caractérisé en ce que** les cations sulfonium répondent à la formule générale SR¹R²R³⁺, où R¹, R² et R³, identiques ou différents, représentent chacun un reste hydrocarbyle ayant de 1 à 12 atomes de carbone, par exemple un groupement alkyle, saturé ou non saturé, cycloalkyle ou aromatique, aryle, alkaryle ou aralkyle, comprenant de 1 à 12 atomes de carbone.

10. Procédé selon la revendication 3, **caractérisé en ce que** le liquide ionique non-aqueux est choisi dans le groupe formé par l'hexafluorophosphate de N-butyl-pyridinium, le tétrafluoroborate de N-éthyl-pyridinium, le fluorosulfonate de pyridinium, le tétrafluoroborate de butyl-3-méthyl-1-imidazolium, l'hexafluoro-antimonate de butyl-3-méthyl-1-imidazolium, l'hexafluorophosphate de butyl-3-méthyl-1-imidazolium, le trifluoroacétate de butyl-3-méthyl-1-imidazolium, le trifluorométhylsulfonate de butyl-3-méthyl-1-imidazolium, le bis(trifluorométhylsulfonyl)amidure de butyl-3-méthyl-1-imidazolium, l'hexafluorophosphate de triméthyl-phénylammonium et le tétrafluoroborate de tétrabutylphosphonium.

11. Procédé selon l'une des revendications 1 à 10, **caractérisé en ce que** les composés du cobalt précurseurs du catalyseur sont choisis dans le groupe formé par les sels et les complexes carbonyles de cobalt, de préférence choisis dans le groupe formé par les acétylacétonates, les carboxylates, le dicobalt-octacarbonyle, l'hydrure de cobalt-tétracarbonyle et les clusters carbonyles.

12. Procédé selon l'une des revendications 1 à 11, **caractérisé en ce que** la concentration du complexe de cobalt dans le liquide ionique est comprise entre 0,1 mmoles par litre et 5 moles par litre et le rapport molaire entre le ligand et le composé du cobalt est compris entre 0,1:1 et 500:1.

13. Procédé selon l'une des revendications 1 à 12, **caractérisé en ce que** la réaction d'hydroformylation est effectuée avec un rapport des pressions partielles de l'hydrogène au monoxyde de carbone de 10:1 à 1:10, à une température comprise entre 30 °C et 200 °C, sous une pression comprise entre 1 MPa et 20 MPa.

14. Procédé selon l'une des revendications 1 à 13, **caractérisé en ce que** le composé oléfiniquement insaturé à hydroformyler est choisi dans le groupe formé par les monooléfines, les dioléfines et les composés oléfiniques comportant un ou plusieurs hétéroatomes.

15. Installation pour la mise en oeuvre du procédé d'hydroformylation selon l'une des revendications 1 à 14, ladite installation comportant :
- au moins un réacteur A1 ;
- au moins une enceinte de dépressurisation (« dépressuriseur ») B1 ;
- et au moins un décanteur B2 pour la décantation de la phase polaire contenant au moins le solvant ionique non-aqueux ionique contenant au moins le catalyseur qui est recyclé au réacteur A1 ;
ainsi que :
- au moins une conduite 1 pour l'introduction de la charge à hydroformyler et du mélange monoxyde de carbone/hydrogène ;
- au moins une conduite 2 pour le transfert de l'effluent du réacteur vers le dépressuriseur B1 ;
- au moins une conduite 3 pour envoyer vers le décanteur B2 le mélange de l'effluent organique et du solvant ionique contenu dans le dépressuriseur B1 ;
- au moins une conduite 6 pour renvoyer vers le réacteur A1 les gaz issus du dépressuriseur B1 ;
- au moins une conduite 5 permettant de renvoyer dans le réacteur A1 la phase polaire contenant au moins le liquide ionique et le catalyseur séparée dans B2 ;
- au moins une conduite 7 permettant de soutirer du décanteur B2 les produits bruts de la réaction.

16. Installation selon la revendication 15 **caractérisée en ce qu'**elle comprend en outre :
- dans la section de séparation, au moins une colonne A2 pour la séparation des produits bruts de la réaction et du composé oléfiniquement insaturé à hydroformyler qui n'a pas réagi ;
- au moins une conduite 4 pour recycler vers le réacteur A1 le composé oléfiniquement insaturé à hydroformyler qui n'a pas réagi séparé dans la colonne A2 ; et
- au moins une conduite 8 permettant d'envoyer les produits sortant en pied de la colonne A2 dans la suite du train de fractionnement des produits.

## Patentansprüche

1. Kontinuierliches Verfahren zur Hydroformylierung in flüssiger Phase von olefinisch ungesättigten Verbindungen, umfassend einen Schritt zur Umsetzung unter Druck, der in Gegenwart von mindestens einer nichtwässrigen, ionischen Flüssigkeit durchgeführt wird, die mindestens ein Salz mit der allgemeinen Formel Q⁺A⁻ umfasst, wobei Q⁺ für ein quartäres Ammonium und/oder ein quartäres Phosphonium und/oder Sulfonium steht, und A⁻ für ein Anion steht, und einen Schritt zum Dekantieren der Endprodukte, **dadurch gekennzeichnet, dass** der Katalysator mindestens einen Komplex aus Cobalt und mindestens einem Liganden umfasst, und dadurch, dass zwischen dem Schritt zum Umsetzen und dem Schritt zum Dekantieren ein dazwischen liegender Schritt zur Druckminderung durchgeführt wird, wobei der Kontakt zwischen den beiden flüssigen Phasen in dem Schritt zur Druckminderung durch mechanisches Rühren aufrecht erhalten wird, wobei der Ligand ausgewählt ist aus der Gruppe bestehend aus:
- Sauerstoff-Liganden, ausgewählt aus der Gruppe bestehend aus Alkoholen, Phenolen, Ethern, Ketonen, Acetalen,
- Schwefel-Liganden, ausgewählt aus der Gruppe bestehend aus Thiolen, Thiophenolen, Thioethern und Disulfiden,
- Stickstoff-Liganden, ausgewählt aus der Gruppe bestehend aus Monoaminen, Di-, Tri- und Polyaminen, Iminen, Diiminen, Pyridinen, Bipyridinen, Imidazolen, Pyrrolen und Pyrazolen und
- Phosphor-Liganden, ausgewählt aus der Gruppe bestehend aus Phosphinen, Polyphosphinen, Oxiden von Phosphinen und Phosphiten,
wobei die Liganden mit ionischen Funktionsgruppen, wie zum Beispiel Sulfonaten, Carboxylaten, Phosphaten, Ammonium- und Phosphoniumverbindungen, substituiert sind oder nicht.

2. Verfahren nach Anspruch 1, wobei der mit Druck beaufschlagte Abfluss des Reaktors in einen Behälter übertragen wird, wo eine Druckminderung bis auf einen Druck kleiner 1 MPa und bevorzugt auf Atmosphärendruck, bei einer Temperatur von höchstens gleich 150 °C und bevorzugt kleiner 60 °C, durchgeführt wird.

3. Verfahren nach einem der Ansprüche 1 oder 2, **dadurch gekennzeichnet, dass** die nicht-wässrige ionische Flüssigkeit ausgewählt ist aus der Gruppe bestehend aus flüssigen Salzen, deren allgemeine Formel Q⁺ A⁻ lautet, wobei Q⁺ für ein quartäres Ammonium und/oder ein quartäres Phosphonium und/oder Sulfonium steht, und A⁻ für jedes Anion steht, dass geeignet ist, bei niedriger Temperatur, das heißt unterhalb von 90 °C, ein flüssiges Salz zu bilden.

4. Verfahren nach Anspruch 3, **dadurch gekennzeichnet, dass** die Anionen A⁻ ausgewählt sind aus den Nitrat-, Sulfat-, Phosphat-, Acetat-, Halogenacetat-, Tetrafluoroborat-, Tetrachoroborat-, Hexafluorophosphat-, Hexafluoroantimonat-, Fluorosulfonat-, Alkylsulfonat-, Perfluoroalkylsulfonat-, Bis(perfluoroalkylsulfonyl)amid-, Arensulfonationen und Arensulfonationen, die mit Halogen- oder Halogenoalkylgruppen substituiert sind,

5. Verfahren nach Anspruch 3, **dadurch gekennzeichnet, dass** die quartären Ammonium- und/oder Phosphoniumkationen den allgemeinen Formeln NR¹R²R³R⁴⁺ und PR¹R²R³R⁴⁺ oder den allgemeinen Formeln R¹R²N=CR³R⁴⁺ et R¹R²P=CR³R⁴⁺ entsprechen, wobei R¹, R², R³ und R⁴, gleich oder verschieden, für Wasserstoff stehen, mit Ausnahme des Kations NH₄⁺, und bevorzugt ein einziger Substituent für Wasserstoff oder Hydrocarbylreste mit 1 bis 30 Kohlenstoffatomen stehen kann.

6. Verfahren nach Anspruch 3, **dadurch gekennzeichnet, dass** die Ammonium- und/oder Phosphoniumkationen von Stickstoff- und/oder Phosphor-Heterocyclen abgeleitet sind, umfassend 1, 2 oder 3 Stickstoffatome und/oder Phosphoratome, wobei die Cyclen aus 4 bis 10 Atomen bestehen.

7. Verfahren nach Anspruch 3, **dadurch gekennzeichnet, dass** die quartären Ammonium- und Phosphoniumkationen jeweils den folgenden Formeln entsprechen:
R¹R²⁺N=CR³-R⁵-R³C=N⁺R¹R²
und
R¹R²+p=CR³-R⁵-R³C=P⁺R¹R²
wobei R¹, R² und R³, gleich oder verschieden, wie oben definiert sind und R⁵ für einen Alkylen- oder Phenylenrest steht.

8. Verfahren nach Anspruch 3, **dadurch gekennzeichnet, dass** die Ammonium- und/oder Phosphoniumkationen ausgewählt sind aus der Gruppe bestehend aus N-Butylpyridinium, N-Ethylpyridinium, Pyridinium, Ethyl-3-methyl-1-imidazolium, Butyl-3-methyl-1-imidazolium, Hexyl-3-methyl-1-imidazolium, Butyl-3-dimethyl-1,2-imidazolium, Diethylpyrazolium, N-Butyl-N-methylpyrrolidinium, Trimethylphenylammonium, Tetrabutylphosphonium und Tributyltetradecylphosphonium.

9. Verfahren nach Anspruch 3, **dadurch gekennzeichnet, dass** die Sulfoniumkationen der allgemeinen Formel SR¹R²R³⁺ entsprechen, worin R¹, R² und R³, gleich oder verschieden, jeweils für einen Hydrocarbylrest mit 1 bis 12 Kohlenstoffatomen, zum Beispiel eine gesättigte oder ungesättigte Alkylgruppe, Cycloalkylgruppe oder aromatische Gruppe, Aryl-, Alkaryl- oder Aralkylgruppe, umfassend 1 bis 12 Kohlenstoffatome, stehen.

10. Verfahren nach Anspruch 3, **dadurch gekennzeichnet, dass** die nicht-wässrige ionische Flüssigkeit ausgewählt ist aus der Gruppe bestehend aus N-Butylpyridinium-hexafluorophosphat, N-Ethylpyridiniumtetrafluoroborat, Pyridiniumfluorosulfonat, Butyl-3-methyl-1-imidazolium-tetrafluoroborat, Butyl-3-methyl-1-imidazolium-hexafluoroantimonat, Butyl-3-methyl-1-imidazolium-hexafluorophosphat, Butyl-3-methyl-1-imidazolium-trifluoroacetat, Butyl-3-methyl-1-imidazolium-trifluoromethylsulfonat, Butyl-3-methyl-1-imidazolium-bis(trifluoromethylsulfonyl)amid, Trimethylphenylammoniumhexafluorophosphat und Tetrabutylphosphonium-tetrafluoroborat.

11. Verfahren nach einem der Ansprüche 1 bis 10, **dadurch gekennzeichnet, dass** die Katalysatorvorläufer-Cobaltverbindungen ausgewählt sind aus der Gruppe bestehend aus Salzen und Cobaltcarbonylkomplexen, bevorzugt ausgewählt aus der Gruppe bestehend aus Acetylacetonaten, Carboxylaten, Dicobaltoctacarbonyl, Cobalttetracarbonylhydrid und Carbonylclustern.

12. Verfahren nach einem der Ansprüche 1 bis 11, **dadurch gekennzeichnet, dass** die Konzentration des Cobaltkomplexes in der ionischen Flüssigkeit im Bereich zwischen 0,1 mmol je Liter und 5 mol je Liter liegt und dass das Molverhältnis zwischen dem Liganden und der Cobaltverbindung im Bereich zwischen 0,1:1 und 500:1 liegt.

13. Verfahren nach einem der Ansprüche 1 bis 12, **dadurch gekennzeichnet, dass** die Hydroformylierungsreaktion mit einem Wasserstoff-zu-Kohlenmonoxid-Partialdruckverhältnis von 10:1 bis 1:10, bei einer Temperatur im Bereich zwischen 30 °C und 200 °C, unter einem Druck im Bereich zwischen 1 MPa und 20 MPa durchgeführt wird.

14. Verfahren nach einem der Ansprüche 1 bis 13, **dadurch gekennzeichnet, dass** die zu hydroformylierende olefinisch ungesättigte Verbindung ausgewählt ist aus der Gruppe bestehend aus Monoolefinen, Diolefinen und olefinischen Verbindungen, die ein oder mehrere Heteroatome umfassen.

15. Anlage zur Durchführung des Hydroformylierungsverfahrens nach einem der Ansprüche 1 bis 14, wobei die Anlage Folgendes umfasst:
- mindestens einen Reaktor A1;
- mindestens einen Behälter zur Druckminderung("Druckminderer") B1;
- und mindestens einen Dekanter B2 zum Dekantieren der polaren Phase, die mindestens das ionische, nicht-wässrige, ionische Lösemittel enthält, das mindestens den Katalysator enthält, der zum Reaktor A1 zurückgeführt wird;
sowie:
- mindestens eine Leitung 1 zum Einführen des zu hydroformylierenden Einsatzes und des Kohlenmonoxid-/Wasserstoff-Gemischs;
- mindestens eine Leitung 2 zum Übertragen des Reaktorabflusses in den Druckminderer B1;
- mindestens eine Leitung 3 zum Schicken zum Dekanter B2 des Gemischs aus dem organischen Abfluss und dem ionischen Lösemittel, das in dem Druckminderer B1 enthalten ist;
- mindestens eine Leitung 6 zum Zurückschicken in den Reaktor A1 der aus dem Druckminderer B1 stammenden Gase;
- mindestens eine Leitung 5, die es ermöglicht, die polare Phase, die mindestens die ionische Flüssigkeit und den in B2 abgetrennten Katalysator enthält, in den Reaktor A1 zurückzuschicken;
- mindestens eine Leitung 7, die es ermöglicht, aus dem Dekanter B2 die Reaktionsrohprodukte abzuziehen.

16. Anlage nach Anspruch 15, **dadurch gekennzeichnet, dass** sie ferner Folgendes umfasst:
- im Trennabschnitt, mindestens eine Säule A2 zum Trennen der Reaktionsrohprodukte und der zu hydroformylierenden, olefinisch ungesättigten Verbindung, die nicht umgesetzt wurde;
- mindestens eine Leitung 4 zum Zurückführen in den Reaktor A1 der zu hydroformulierenden, olefinisch ungesättigten Verbindung, die nicht umgesetzt wurde, die in der Säule A2 getrennt wurde; und
- mindestens eine Leitung 8, die es ermöglicht, die Produkte, die am Fuß der Säule A2 austreten, in die Fortsetzung des Fraktionierungsablaufs zu schicken.

## Claims

1. A continuous process for the liquid phase hydroformylation of olefinically unsaturated compounds, comprising a step of reacting under pressure and in the presence of at least one non-aqueous ionic liquid comprising at least one salt with general formula Q⁺ A- in which Q⁺ represents a quaternary ammonium cation and/or a quaternary phosphonium cation and/or a quaternary sulphonium cation and A⁻ represents an anion, and a step for decanting the final products, said process being **characterised in that** the catalyst comprises at least one complex of cobalt with at least one ligand, and that between the reaction step and the decanting step, an intermediate step is carried out in which the effluent of the hydroformylation reaction is depressurised under mechanic stirring, said ligand being selected from the
- oxygen-containing ligands selected from the group consisting of alcohols, phenols, ethers, ketones and acetals,
- ulphur-containing ligands selected from the group consisting of thiols, thiophenols, thioethers and disulphides,
- nitrogen-containing ligands selected from the group consisting of monoamines, di-, tri- and poly-amines, imines, diimines, pyridines, bipyridines, imidazoles, pyrroles and pyrazoles, and
- phosphorus-containing ligands selected from the group consisting of phosphines, polyphosphines, phosphine oxides and phosphites
said ligand being substituted by ionic functional groups such as sulphonates, carboxylates, phosphates, ammonium or phosphonium compounds.

2. A process according to claim 1, in which the effluent from the pressurised reactor is transferred into a vessel in which it is depressurised to a pressure of less than 1 MPa and preferably to atmospheric pressure, to a temperature of at most 150°C and preferably below 60°C.

3. A process according to claim 1 or 2 **characterised in that** the non-aqueous ionic liquid is selected from the group consisting of liquid salts with general formula Q⁺A⁻ in which Q⁺ represents a quaternary ammonium and/or a quaternary phosphonium and/or a quaternary sulphonium cation and A⁻ represents any anion that is capable of forming a liquid salt at low temperatures, i.e., below 90°C

4. A process according to claim 3, **characterized in that** anions A⁻ are selected from the group consisting of nitrate, sulphate, phosphate, acetate, halogenoacetates, tetrafluoroborate, tetrachloroborate, hexafluorophosphate, hexafluoroantimonate, fluorosulphonate, alkylsulphonates, perfluoroalkylsulphonates, bis(perfluoroalkyl-sulphonyl)amides, arenesulphonates and arenesulphonates substituted with halogen or halogenoalkyl groups.

5. A process according to claim 3, **characterised in that** the quaternary ammonium and/or phosphonium cations Q⁺ have general formulae NR¹R²R³R⁴⁺ and PR¹R²R³R⁴⁺, or general formulae R¹R²N = CR³R⁴⁺ and R¹R²P = CR³R⁴⁺ in which R¹, R², R³ and R⁴, which may be identical or different, represent hydrogen with the exception of the cation NH₄⁺ and preferably a single substituent can represent hydrogen or hydrocarbyl residues containing 1 to 30 carbon atoms.

6. A process according to claim 3, **characterised in that** the ammonium and/or phosphonium cations are derived from nitrogen-containing and/or phosphorus-containing heterocycles containing 1, 2 or 3 nitrogen and/or phosphorus atoms in which the cycles are constituted by 4 to 10 atoms.

7. A process according to claim 3, **characterised in that** the quaternary ammonium and phosphonium cations respectively satisfy the following formulae:
R¹R²⁺N = CR³-R⁵-R³C = N⁺R¹R²
and
R¹R²⁺P = CR³-R⁵-R³C = P⁺R¹R²
in which R¹, R² and R³, which may be identical or different, are as defined above, and R⁵ represents an alkylene or phenylene radical.

8. A process according to claim 3, **characterised in that** the ammonium and/or phosphonium cations are selected from the group consisting of N-butylpyridinium, N-ethylpyridinium, pyridinium, 3-ethyl-1-methylimidazolium, 3-butyl-1-methylimidazolium, 3-hexyl-1-methylimidazolium, 3-butyl-1,2-dimethylimidazolium, diethylpyrazolium, N-butyl-N-methylpyrrolidinium, trimethylphenylammonium, tetrabutylphosphonium and tributyl-tetradecylphosphonium.

9. A process according to claim 3, **characterised in that** the sulphonium cations have general formula SR¹R²R³⁺, in which R¹, R² and R³, which may be identical or different, each represent a hydrocarbyl radical containing 1 to 12 carbon atoms, for example an alkyl, saturated or unsaturated, cycloalkyl or aromatic, aryl, alkaryl or aralkyl group containing 1 to 12 carbon atoms.

10. A process according to claim 3, **characterised in that** the non-aqueous ionic liquid is selected from the group consisting of N-butylpyridinium hexafluorophosphate, N-ethyl pyridinium tetrafluoroborate, pyridinium fluorosulfonate, 3-butyl-1-methylimidazolium tetrafluoroborate, 3-butyl-1-methylimidazolium hexafluoroantimonate, 3-butyl-1-methylimidazolium hexafluorophosphate, 3-butyl-1-methylimidazolium trifluoroacetate, 3-butyl-1-methylimidazolium trifluoromethylsulphonate, 3-butyl-1-methylimidazolium bis(trifluoromethylsulfonyl)amide, trimethylphenylammonium hexafluorophosphate and tetrabutylphosphonium tetrafluoroborate.

11. A process according to one of claims 1 to 10, **characterised in that** catalyst cobalt compound precursors are selected from the group consisting of cobalt salts and carbonyl complexes, preferably selected from the group consisting of acetylacetonates, carboxylates, dicobalt-octacarbonyl, cobalt-tetracarbonyl hydride and carbonyl clusters.

12. A process according to one of claims 1 to 11, **characterised in that** the concentration of the cobalt complex in the ionic liquid is in the range 0.1 mmoles per litre of ionic liquid to 5 moles per litre, and the mole ratio between the ligand and the cobalt compound is in the range 0.1: 1 to 500: 1.

13. A process according to one of claims 1 to 12, **characterised in that** the hydroformylation reaction is carried out with a ratio of the partial pressures of hydrogen to carbon monoxide of 10:1 to 1:10, at a temperature in the range 30°C to 200°C, and at a pressure in the range 1 MPa to 20 MPa.

14. A process according to one of claims 1 to 13, **characterised in that** the olefinically unsaturated compound to be hydroformylated is selected from the group consisting of mono-olefins, di-olefins and olefinic compounds containing one or more heteroatoms.

15. An apparatus for carrying out the hydroformylation process according to one of claims 1 to 14, said apparatus comprising:
- at least one reactor A1;
- at least one depressurisation vessel ("depressuriser") B1;
- at least one decanter B2 to decant the polar phase containing at least the non-aqueous ionic solvent containing at least the catalyst which is recycled to reactor A1;
linked with one another by:
- at least one line 1 for introducing feed to be hydroformylated and the carbon monoxide/hydrogen mixture;
- at least one line 2 for transferring the effluent from the reactor to the depressurizer B1;
- at least one line 3 for sending the mixture of the organic effluent and the ionic solvent contained in the depressuriser B1 to the decanter B2;
- at least one line 6 for returning the gas from the depressuriser B1 to the reactor A1;
- at least one line 5 for returning the polar phase containing at least the ionic liquid and the catalyst separated in B2 to the reactor A1;
- at least one line 7 which can withdraw the as synthesised reaction products from the decanter B2.

16. An apparatus according to claim 15 further comprising:
• in a separation section, at least one column A2 for separating the crude reaction products from the unreacted olefinically unsaturated compound to be hydroformylated;
• at least one line 4 for recycling the unreacted olefinically unsaturated compound to be hydroformylated separated in column A2 to the reactor A1; and
• at least one line 8 designed to send the products leaving from the foot of the column A2 to the remainder of the product fractionation train.
